Europäisches Patentamt

**European Patent Office**

**Office européen des brevets**

(19)

(11) Numéro de publication: **0 384 034**
**A2**

(12) **DEMANDE DE BREVET EUROPEEN**

(21) Numéro de dépôt: **89123637.4**

(22) Date de dépôt: **21.12.89**

(51) Int. Cl.5: **A61K 7/32, A61K 7/00,**
**A61K 7/46**

(30) Priorité: **21.02.89 CH 598/89**

(43) Date de publication de la demande:
**29.08.90 Bulletin 90/35**

(84) Etats contractants désignés:
**CH DE ES FR GB IT LI**

(71) Demandeur: **FIRMENICH SA**
**1, route des Jeunes**
**CH-1211 Genève 8(CH)**

(72) Inventeur: **Holzner, Günther**
**15, chemin des Palettes**
**CH-1212 Grand-Lancy(CH)**
Inventeur: **Moulin, Daniel**
**1, chemin du Fief de Chapitre**
**CH-1213 Petit-Lancy(CH)**

(74) Mandataire: **Salvadori, Giuseppe, Dr.**
**c/o Firmenich S.A. Case Postale 239**
**CH-1211 Genève 8(CH)**

(54) **Composition parfumante à action désodorisante ou antiperspirante.**

(57) La composition contient une base désodorisante ou antiperspirante active, une base parfumante sous forme d'émulsion aqueuse ou sous forme microencapsulée, ladite base parfumante étant combinée avec un agent filmogène et un agent émulsifiant. L'agent filmogène contient soit essentiellement de la polyvinylpyrrolidone, soit un mélange de celle-ci avec un ou plusieurs composés choisis parmi l'acétate de polyvinyle, l'alcool polyvinylique, les dextrines, l'amidon, naturel ou modifié, les gommes végétales, les pectines, les xanthanes, la carboxyméthylcellulose, la méthylcellulose, l'hydroxyéthylcellulose et les lipohétéropolysaccharides. L'agent émulsifiant est choisi parmi les mono- ou diglycérides d'acides gras, les esters dérivés de la combinaison d'acides gras avec le sorbitol ou un saccharide, ou leurs dérivés alcoxylés, ou un ester de l'acide tartrique, citrique, ascorbique ou lactique.

Ladite composition présente l'avantage de libérer le constituant volatile du parfum au moment désiré sous l'action d'une source d'humidité, la sueur en particulier. Elle présente en outre l'avantage de donner lieu à une réencapsulation in situ, par exemple sur la peau même, de constituants actifs dans la phase de séchage. Elle convient spécialement à la manufacture d'articles destinés aux soins corporels tels les désodorisants et antiperspirants façonnés sous forme de bâtonnets, de dispositifs à bille, de smooth-on ou d'aérosols et vaporisateurs à pression.

EP 0 384 034 A2

## Composition parfumante à action désodorisante ou antiperspirante

La présente invention a trait à une composition parfumante à action désodorisante ou antiperspirante destinée aux soins corporels, contenant une base désodorisante ou antiperspirante active, et une base parfumante sous forme d'émulsion aqueuse ou sous forme microencapsulée, cette base parfumante étant combinée avec un agent filmogène et un agent émulsifiant.

Comme il est décrit dans la demande de brevet européen n° 279 328, appartenant au même titulaire que la présente demande, les compositions parfumantes de ce type présentent l'avantage de permettre le contrôle de l'activation et de la diffusion du parfum dans le temps. Les informations contenues dans ladite demande sont incluses ici par référence. Grâce à un choix particulier des ingrédients entrant dans la composition, notamment des agents filmogène et émulsifiant, il est possible de provoquer un phénomène réversible de "réencapsulation" des ingrédients désodorisants actifs, de telle sorte que plusieurs activations successives peuvent avoir lieu sur l'épiderme même sans que l'utilisateur ait à procéder à de nouvelles applications. La réencapsulation a lieu in situ lors de la phase de séchage de l'épiderme qui suit la période de transpiration.

Or nous avons maintenant découvert que l'emploi d'un ingrédient en particulier, à savoir la polyvinylpyrrolidone, en tant que composant de l'agent filmogène, permettait d'obtenir des compositions désodorisantes ou antiperspirantes présentant des avantages par rapport à celles décrites dans la demande de brevet susmentionnée. Ceci résulte du fait que la polyvinylpyrrolidone est soluble dans l'alcool, pouvant former des solutions parfaitement limpides, particulièrement avantageuses pour des applications telles que sticks et roll-on transparents, appréciés pour leurs qualités esthétiques. Par ailleurs, l'emploi de la polyvinylpyrrolidone, en tant que composant de l'agent filmogène, dans des compositions destinées à d'autres types d'articles ou dispositifs désodorisants ou antiperspirants s'est révélé souvent préférentiel car il permettait d'augmenter le rapport alcool/eau de la composition et d'accélérer ainsi le séchage de cette dernière lors de l'application.

La présente invention a donc pour objet une composition parfumante à action désodorisante ou antiperspirante destinée aux soins corporels, contenant une base désodorisante ou antiperspirante active, et une base parfumante sous forme d'émulsion aqueuse ou sous forme microencapsulée, ladite base parfumante étant combinée avec un agent filmogène et un agent émulsifiant, la composition étant caractérisée en ce que l'agent filmogène contient de la polyvinylpyrrolidone.

La composition selon l'invention concilie la nécessité de protéger les principes actifs du parfum de l'action du milieu, particulièrement agressif dans le cas d'antiperspirants, et le souhait de prolonger la diffusion de ce parfum. Cette double action résulte du phénomène expliqué plus haut. Lors de l'application sur la peau, la composition est d'abord retenue à la surface par adhésion de l'émulsion, lorsque la base parfumante est employée sous forme d'émulsion aqueuse, et ce grâce à l'effet de liaison exercé par l'agent filmogène. Sous l'effet du séchage qui s'ensuit, et qui s'effectue par simple exposition à l'air de la peau ainsi traitée, effet favorisé par la chaleur du corps, la base parfumante active est retenue sous forme de gouttelettes microscopiques enrobées d'une couche protectrice hydrosoluble constituée par cet agent désormais sec.

Il s'agit en l'occurrence d'un système simple qui ne nécessite pas la mise en application de dispositifs spéciaux pour son emploi. Tout système conventionnel d'utilisation courante en cosmétique, et utilisé couramment pour l'application de désodorisants et antiperspirants, peut être employé. On peut mentionner à cet effet les crèmes, les roll-on, les smooth-on ou encore les poudres.

Il en va de même lorsque la base parfumante active est employée sous forme microencapsulée, généralement en suspension dans un solvant alcoolique. La base parfumante, combinée préalablement à un agent filmogène et à un agent émulsifiant, est atomisée selon les techniques usuelles dans une tour de type "spray-drier". Les microcapsules obtenues renfermant la base parfumante sont mélangées à une base désodorisante ou antiperspirante et ensuite suspendues dans une base constituée essentiellement par des cires, selon la technique employée pour la fabrication des sticks, ou dans un mélange propulseur pour la fabrication d'aérosols.

Selon un mode d'exécution de l'invention, l'agent filmogène est constitué essentiellement par la polyvinylpyrrolidone. Selon un autre mode d'exécution plus économique, l'agent filmogène contient, outre la polyvinylpyrrolidone, un ou plusieurs composés choisis parmi l'acétate de polyvinyle, l'alcool polyvinylique, les dextrines (naturelles ou modifiées), l'amidon (naturel ou modifié), les gommes végétales, les alginates, les carragénanes, les pectines, les xanthanes, ou encore les dérivés de la cellulose telles par exemple la carboxyméthylcellulose, la méthylcellulose et l'hydroxyéthylcellulose. Il s'agit de composés qui peuvent être définis sous le terme générique de "gommes" (voir à cet effet la définition donnée dans Kirk-Othmer, Encyclopedia of Chemical Technology, 2ème édition, vol. 10, p.741).

Nous trouvons donc sous ce terme des gommes naturelles, tels par exemple la gomme arabique, des extraits d'algues, par exemple l'agar, les carragénanes, le furcellarane et des gommes modifiées ou semi-synthétiques. Il s'agit de dérivés de la cellulose et de l'amidon ainsi que de gommes de fermentation microbienne, tels les hétéropoly-saccharides, par exemple les biopolymères connus sous le nom d'emulsan (voir demande de brevet européen n° 178'443, publiée le 23.04.86).

A titre d'agent émulsifiant, on peut utiliser des mono- ou diglycérides d'acides gras, les esters dérivés de la combinaison d'acides gras avec le sorbitol ou un monosaccharide, ou leurs dérivés alcoxylés, ou un ester de l'acide tartrique, citrique, ascorbique ou lactique.

La composition suivant l'invention contient également une base parfumante. Aux termes de la présente invention, on entend par "base parfumante" toute substance ou mélange de substances parfumantes, tant à l'état isolé qu'en solution ou suspension, dans leurs diluants, dissolvants ou coingrédients habituels. Ce terme comprend en particulier des solutions organiques généralement non-miscibles à l'eau dotées d'une tension de vapeur appréciable. De telles bases parfumantes peuvent être constituées par des composés appartenant à des classes chimiques distinctes et comprennent par exemple des esters, des éthers, des alcools, des aldéhydes, des cétones, des acétals, des nitriles, des hydrocarbures terpéniques, des composés hétérocycliques azotés ou soufrés ainsi que des huiles essentielles d'origine naturelle. Le choix particulier de la base parfumante dépend de l'effet odorant recherché, de la nature du produit que l'on désire parfumer et bien entendu du goût et préférence du parfumeur créateur.

Des exemples typiques de composés parfumants utiles sont donnés dans la littérature et, à cet effet, on peut citer S. Arctander, Perfume and Flavor Chemicals, Montclair, N.J. (USA), 1969.

La composition selon l'invention peut également contenir des agents bactéricides, germicides ou bactériostatiques.

Quoiqu'il soit difficile de définir une gamme précise de concentration de ses constituants, la composition parfumante de l'invention contient à titre préférentiel (en poids)

a. 2 à 20% d'agent filmogène,

b. 0,1 à 10% d'agent émulsifiant,

c. 0,1 à 5% de base parfumante,

d. 0,1 à 50% de base désodorisante ou antiperspirante active, le restant étant représenté par de l'eau, des dissolvants inertes et/ou des excipients, le cas échéant des agents, germicides ou bactériostatiques.

Par base désodorisante, on entend une substance capable de masquer l'odeur corporelle et d'inhiber la prolifération des bactéries responsables de la dégradation de la sueur. Bon nombre de produits bactéricides et bactériostatiques sont connus et utilisés à cet effet. A titre d'exemple, on peut mentionner l'hexachlorophène, le dichlorophénol, le trichlorosalicylanilide, le tribromosalicylanilide (TBS), le tétrachlorosalicylanilide (TCSA), le trichlorocarbanilide (TCC) et l'Irgasan (marque enregistrée) DP-300 (Ciba-Geigy).

A titre de base antiperspirante, on peut utiliser de préférence des sels d'aluminium, par exemple le chlorhydrate d'aluminium mentionné plus haut. Différentes compositions sont proposées sur le marché en tant que produit de base antiperspirante: le Chlorhydrol, le Chloracel et le Rezal (marques enregistrées de Reheis Chem. Co., USA) en sont des exemples. Il s'agit de sels complexes d'aluminium ou aluminium et zirconium. D'autres bases antiperspirantes sont décrites dans la littérature spécialisée (voir par exemple: Herbert P. Fiedler, Der Schweiss, Editio Kantor KG, Aulendorf i. Württ., RFA).

La composition parfumante de l'invention convient tout particulièrement à la manufacture d'articles destinés aux soins corporels. Ces derniers peuvent se présenter sous une multitude de formes différentes. On a eu l'occasion de mentionner plus haut les bâtonnets (sticks), les roll-on (dispositifs à bille), les smooth-on, ou encore les aérosols ou les vaporisateurs à pression mécanique ou manuelle. On a également cité l'emploi préférentiel de cette composition pour réaliser des sticks et roll-on transparents.

La composition selon l'invention est obtenue par mélange de ses différents ingrédients au moyen d'appareillage conventionnel. La technique de mélange est en soi connue et toute explication détaillée est ici superflue. On précisera que la méthode dépend essentiellement de l'article final que l'on désire façonner. C'est ainsi que par exemple, si l'on désire préparer une composition antiperspirante destinée à être employée à l'aide d'un dispositif à bille du type roll-on, l'on peut procéder de la sorte.

On verse à température ambiante la poudre constituée par l'agent filmogène, par exemple un mélange de polyvinylpyrrolidone et maltodextrines, dans la quantité requise d'eau déminéralisée. Après dissolution complète, on ajoute à la solution obtenue la base antiperspirante et le tout est brassé et chauffé à 70°C puis, à cette même température, on ajoute l'agent émulsifiant en se servant d'un mélangeur homogénéisateur. Après quelques minutes de brassage, on refroidit le mélange à température ambiante et on y ajoute la base parfumante à environ 40°C. La masse visqueuse parfumée est enfin versée dans des récipients à bille du type roll-on.

Si l'on désire préparer une composition antiperspirante humide destinée à être employée à l'aide d'un dispositif de vaporisation à pression mécanique ou manuelle, on procèdera de préférence ainsi.

On verse à température ambiante l'agent filmogène en poudre dans l'eau ou dans un mélange éthanol-eau et on brasse pendant environ 1 heure jusqu'à dissolution complète. A la solution résultante, on ajoute sous agitation la base antiperspirante active (par exemple de l'hydroxychlorure d'aluminium ou de zirconium), puis la base parfumante préalablement mélangée à un émulsifiant. Le mélange est ensuite versé dans des récipients vaporisateurs.

L'invention est illustrée mais non limitée par les exemples suivants dans lesquels les températures sont indiquées en degrés centigrades et les abréviations ont le sens usuel dans l'art.

## Exemple 1

### Composition antiperspirante pour roll-on

Une composition antiperspirante de type crémeux destinée à être incorporée dans un distributeur à bille de type roll-on a été préparée en ajoutant par petites portions un mélange constitué par

3,00 g de Luviskol (marque enregistrée) K 30 (polyvinylpyrrolidone poudre) 1)

5,90 g de Glucidex 21 (maltodextrine DE 20-23) 2)

1,00 g de Nadex 722 (maltodextrine DE 9-12) 3)

0,10 g d'alginate de sodium

à 65 g d'eau déminéralisée.

Le mélange a été brassé pendant 2 heures jusqu'à dissolution complète des ingrédients, puis 20 g de Locron (Locron L, hydroxychlorure d'aluminium, solution à 50% Hoechst AG) y ont été ajoutés. Après avoir porté la température du mélange à 70°, on a ajouté 4 g d'Emulgade 1000 NI (cire autoémulsionnante, nonionogène, Henkel AG) sous vigoureuse agitation à l'aide d'un homogénéisateur Ultra Turrax. Après quelques minutes d'homogénéisation, on a refroidi à température ambiante et ajouté 1 g de parfum (Vera 72276/B, type eau-de-Cologne, Firmenich SA, Genève) à environ 40°. Le mélange obtenu a été enfin versé dans des emballages distributeurs de type roll-on.

## Exemple 2

### Composition antiperspirante pour roll-on

Une composition antiperspirante de type crémeux destinée à être incorporée dans un distributeur à bille de type roll-on a été préparée en ajoutant par petites portions 2 g de Luviskol K 30 (BASF) et 8 g de Capsul (amidon de maïs modifié, National Starch) à 63,95 g d'eau déminéralisée. Après 2 h de brassage, on a ajouté un mélange constitué par:

0,10 g de Tween 20 (sorbitanmonolaurate éthoxylé ICI, Atlas)

1,00 g de Triton CG 110 (alkylglucoside, Röhm & Haas)

et 0,05 g d'Emulsan (Petroferm, biopolymère),

puis 10 g de Locron L (Hoechst AG) y ont été ajoutés et le tout a été chauffé à 70°. A cette température, on a additionné 4 g d'Emulgade 1000 NI (Henkel AG) sous vigoureuse agitation à l'aide d'un appareil homogénéisateur. Après quelques minutes de brassage, on a refroidi à température ambiante et 1 g de parfum (Surf 635.040 E, de type aldéhydé, fleuri, vert, Firmenich SA, Genève) y a été ajouté. Le mélange

1) BASF   AG

2) Roquette   Frères

3) Grain   Processing   Corp.

obtenu a été enfin versé dans des récipients distributeurs de type roll-on.

<div align="center"><u>Exemple 3</u></div>

Compositions antiperspirantes pour roll-on

Des compositions antiperspirantes d'aspect limpide destinées à être incorporées dans des distributeurs à bille de type roll-on ont été préparées avec les ingrédients suivants (parties en poids):

|  | a | b |
|---|---|---|
| I. Eau déminéralisée | 11,8 | 11,8 |
| II. Natrosol 250 LR [1] | 1,2 | - |
| Rezal 67 (solution à 40%) [2] | 45,0 | 45,0 |
| III. Luviskol (marque enregistrée) K 30 [3] | 1,5 | 2,7 |
| Ethanol 95° | 38,0 | 38,0 |
| IV. Cremophor RH 40 [4] | 1,5 | 1,5 |
| Parfum [5] | 1.0 | 1.0 |
|  | 100,0 | 100,0 |

1) hydroxyéthylcellulose, Hercules Co.
2) complex zirconium chlorhydrate, Reheis Chem. Co.
3) voir exemple 1
4) huile de ricin hydrogéné et éthoxylé, BASF AG
5) Gabriela 230 183 de type vert, floral, fruité, Firmenich SA, Genève

On a versé la partie II dans de l'eau et on a brassé jusqu'à ce que la solution soit complètement limpide. On a ensuite dissous le Luviskol K 30 dans l'éthanol pour préparer la partie III et émulsionné le parfum dans le Cremophor RN 40 pour préparer la partie IV. La partie III a été ensuite ajoutée peu à peu à la solution en agitant continuellement jusqu'à ce que ce soit parfaitement homogène. La partie IV a enfin été ajoutée et bien mélangée et le tout a été versé dans des récipients distributeurs roll-on. Toutes les opérations se sont déroulées à température ambiante.

<div align="center"><u>Exemple 4</u></div>

Composition antiperspirante pour smooth-on

Une composition antiperspirante destinée à être incorporée dans un récipient de type smooth-on a été préparée à l'aide des ingrédients suivants:

| | |
|---|---|
| I. Eau déminéralisée | 37,0 |
| II. Luviskol K 30 [1] | 2,0 |
| Glucidex 21 [2] | 7,0 |
| Nadex [3] | 1,0 |
| III. Locron L [4] | 40,0 |
| IV. Emulgade 1000 NI [5] | 8,0 |
| Arlacel 165 [6] | 4,0 |
| V. Parfum [7] | 1,0 |
| | 100,0 |

1) voir exemple 1
2) - idem -
3) - idem -
4) - idem -
5) - idem -
6) glycérylstéarate + PEG 1000 stéarate, ICI Atlas
7) Ambrosia UN 110.381/B, de type fleuri, musqué, boisé,
Firmenich SA, Genève

La partie II a été dissoute dans l'eau déminéralisée et à la solution obtenue on a ajouté la partie III, puis l'on a chauffé à 70°. Au mélange résultant, on a ensuite ajouté la partie IV préalablement chauffée à 70°. Après brassage énergique dans un appareil homogénéisateur, on a refroidi le tout et le parfum y a été ajouté à 40° environ sous agitation.

## Exemple 5

### Composition antiperspirante pour spray à pression

Une composition antiperspirante destinée à être employée au moyen d'un appareil distributeur comportant un système gicleur mécanique ou à pression manuelle ("pumpspray" ou "squeeze bottle") a été préparée à l'aide des ingrédients suivants:

| | |
|---|---|
| I. Eau déminéralisée | 39,0 |
| II. Luviskol K 30 [1] | 2,0 |
| Glucidex 21 [2] | 8,0 |
| III. Locron L [3] | 20,0 |
| 1,3-Butylène-glycol | 1,5 |
| Ethanol 95° | 26,5 |
| IV. Parfum [4] | 1,0 |
| Cremophor RH 40 [5] | 2,0 |
| | 100,0 |
| 1) 2) 3) voir exemple 1 | |

4) Diabolo UN 110.382/B, de type fleuri, vert, hespéridé,
Firmenich SA, Genève
5) voir exemple 3

La partie II a été versée dans l'eau déminéralisée et brassée jusqu'à l'obtention d'une solution claire (1 h). III et IV ont été ensuite ajoutées successivement sous agitation et le mélange résultant a été versé dans des récipients gicleurs.

## Exemple 6

Composition antiperspirante pour spray aérosol

Une composition parfumante de base, destinée à être incorporée dans un produit antiperspirant appliqué au moyen d'un distributeur de type "spray", a été préparée par atomisation du mélange suivant:

|  | a |
|---|---|
| Eau | 49,0 |
| Glucidex 21 [1] | 30,0 |
| Nadex [2] | 4,0 |
| Luviskol K 30 [3] | 6,0 |
| Alginate de sodium | 0,8 |
| Capsul [4] | - |
| Tween 20 | 0,2 |
| Parfum [5] | 10,0 |
|  | 100,0 |
| 1) 2) 3) voir exemple 1 | |

4) amidon de maïs modifié, National Starch
5) Surf 635.040 E, Firmenich SA, Genève

Pour effectuer l'atomisation, on a recours à un appareil de type Leaflash (CCM Sulzer):

| débit de l'émulsion | 50 kg/h |
|---|---|
| air de séchage | 320 m$^3$/h à 350° C et 0,45 bar |

La composition de base obtenue a ensuite servi à la préparation de compositions antiperspirantes pour sprays aérosol par mélange avec les ingrédients suivants:

| | |
|---|---|
| I. Composition de base (a) | 3,00 |
| Hydroxychlorure d'aluminium en poudre micronisé (Hoechst) | 4,20 |
| Allantoinate d'hydroxychlorure d'aluminium (Merck) | 0,50 |
| Myristate d'isopropyle | 6,85 |
| Aerosil 200 (Degussa) | 0,25 |
| Irgasan DP 300 (Ciba-Geigy) | 0,20 |
| Propulseur 11 [1] | 50,00 |
| II. Propulseur 12 [2] | 5,00 |
| Propane/Butane [3] | 30,00 |
| | 100,00 |

1) monofluorotrichlorométhane
2) difluorodichlorométhane
3) mélange 3,7 bar

On a mélangé préalablement la partie I afin d'obtenir une suspension homogène et on a ensuite versé le tout dans un récipient aérosol avec adjonction de la partie II.

## Exemple 7

Composition antiperspirante pour smooth-on

Une composition antiperspirante pour smooth-on a été préparée à l'aide des ingrédients suivants (parties en poids):

| | |
|---|---|
| I. Alcool cétylique | 9,0 |
| Cire d'abeilles | 4,5 |
| Acide stéarique | 4,5 |
| Finsolv TN [1] | 10,0 |
| Arlacel 165 [2] | 5,4 |
| II. Hydroxychlorure d'aluminium en poudre micronisée [3] | 20,0 |
| Talc | 5,0 |
| III. Dow Corning fluid 345 [4] | 35,6 |
| IV. Composition de base (a) [5] | 6,0 |
| | 100,0 |

1) benzoate d'alcool C12-C15, Finetex
2) voir exemple 4
3) voir exemple 6
4) huile de silicone volatile
5) voir exemple 6

On a chauffé la partie I à 80° jusqu'à fusion complète de tous ses ingrédients et au mélange fondu on a ajouté successivement les parties II, III et IV sous bonne agitation. On a laissé ensuite refroidir à 40-50° et on a versé dans des récipients de type smooth-on.

Exemple 8

Composition antiperspirante pour bâtonnets

Une composition antiperspirante pour bâtonnets secs ("dry sticks") a été préparée à l'aide des ingrédients suivants (parties en poids):

| | |
|---|---|
| I. Octadécanol | 19,0 |
| Arlacel 165 [1] | 1,0 |
| PEG 1000 [2] | 5,0 |
| II. Aerosil 200 [3] | 1,4 |
| Talc | 1,0 |
| Rezal 36 P [4] | 19,0 |
| III. Dow Corning Fluid 345 [5] | 47,6 |
| IV. Composition de base (a) [6] | 6,0 |
| | 100,0 |

1) voir exemple 4
2) polyglycol 1000, Hoechst
3) Degussa
4) complex zirconium chlorhydrate, Reheis Chem. Co.
5) huile de silicone volatile
6) voir exemple 6

8

On a chauffé à 90° la partie I jusqu'à dissolution complète de tous les ingrédients, puis on a arrêté le chauffage et la partie II a été ajoutée au mélange obtenu. Les parties III et IV ont ensuite été ajoutées successivement sous agitation. Le mélange a été enfin versé dans des moules appropriés à 65° environ.

## Exemple 9

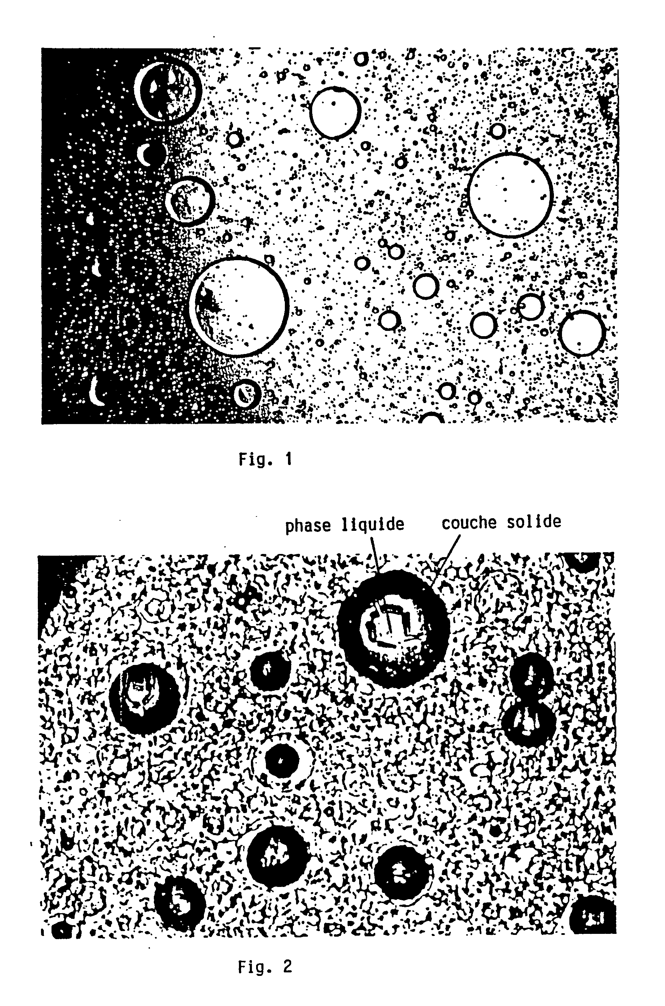

Afin de démontrer l'effet réversible du phénomène de solubilité et "réencapsulation" de la composition selon l'invention, la composition antiperspirante obtenue selon l'exemple 3 a été étalée en couche mince sur un porte-objet et observée au microscope (grossissement 600x). La reproduction de la fig. 1 montre la formation de gouttelettes distinctes d'un diamètre variable. Après 1-2 minutes à l'air, l'émulsion a séché en donnant lieu à la formation d'une membrane solide qui entoure une phase liquide (émulsion) constituée par le parfum (fig. 2).

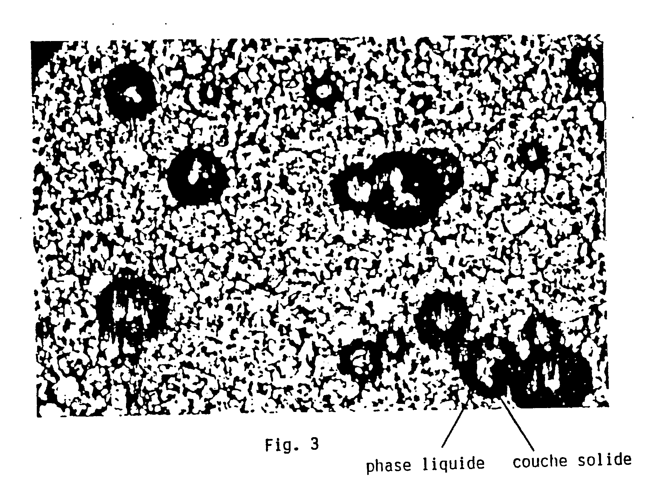

En humidifiant les capsules ainsi formées par l'adjonction de quelques gouttes d'eau, les membranes solides se désagrègent en libérant ainsi la base parfumante liquide qui, au contact direct de l'air, se volatilise en partie dans l'atmosphère environnante. Par un nouveau séchage à l'air, les microcapsules se reforment de sorte que la base parfumante restante est à nouveau entourée, sous forme de gouttelettes, par une membrane solide protectrice (fig. 3).

Un tel phénomène peut être répété plusieurs fois au cours d'une même journée sans qu'on ait observé une altération notable des propriétés du système jusqu'à évaporation complète du parfum.

Un test de stockage prolongé a montré qu'un tel système conserve ses caractères pendant 2 ans au moins.

Le test effectué et décrit ci-dessus tend à montrer l'une des caractéristiques utiles et inattendues de la composition selon l'invention. Utilisée en effet par aspersion directe sur la peau, la composition sèche d'abord pour donner lieu à la formation de microcapsules renfermant la base parfumante. Par l'action de la sueur, ou au contact d'une source d'humidité, la base parfumante est libérée pour être de nouveau encapsulée in situ une fois que la surface de la peau devient sèche. Le dégagement des constituants volatiles de la base, et par voie de conséquence, l'effet masquant et déodorant de celle-ci, est réellement effectif au moment requis ; l'utilisateur lui-même contrôle en quelque sorte physiologiquement un tel dégagement.

## Exemple 10

Une composition antiperspirante, préparée conformément à l'exemple 6, a été appliquée à l'aide d'un spray aérosol dans la région axillaire de 10 sujets mâles d'âge compris entre 21 et 36 ans. L'odeur initiale dégagée par l'épiderme ainsi traité était faible, voire dans certains cas nulle. Après deux heures environ, les sujets ont été soumis à une activité physique intense, telle celle représentée par 20 minutes de jeu de basket. Une évaluation olfactive réalisée à ce moment-là a permis d'établir que le dégagement de parfum était intense. Pendant la période de pose (5 minutes) qui a suivi, le séchage à l'air de la sueur a entraîné une diminution importante de la diffusion du parfum. A la reprise du jeu, le phénomène de transpiration a provoqué à nouveau le dégagement de parfum qui a été nettement perçu par un panel d'évaluation au terme du jeu pour diminuer ou disparaître après quelques minutes de séchage à l'air.

## Revendications

1. Composition parfumante à action désodorisante ou antiperspirante destinée aux soins corporels, contenant une base désodorisante ou antiperspirante active, et une base parfumante sous forme d'émulsion aqueuse ou sous forme microencapsulée, cette base parfumante étant combinée avec un agent filmogène et un agent émulsifiant, la composition étant caractérisée en ce que l'agent filmogène contient de la polyvinylpyrrolidone.

2. Composition parfumante selon la revendication 1, caractérisée en ce que l'agent filmogène est constitué essentiellement par de la polyvinylpyrrolidone.

3. Composition parfumante selon la revendication 1, caractérisée en ce que l'agent filmogène contient, outre la polyvinylpyrrolidone, au moins un composé choisi parmi l'acétate de polyvinyle, l'alcool polyvinylique, les dextrines, l'amidon, naturel ou modifié, les gommes végétales, les pectines, les xanthanes, la carboxyméthylcellulose, la méthylcellulose, l'hydroxyéthylcellulose et les lipohétéropolysaccharides.

4. Composition parfumante selon l'une des revendications précédentes, caractérisée en ce que l'agent émulsifiant est choisi parmi les mono- ou diglycérides d'acides gras, les esters dérivés de la combinaison d'acides gras avec le sorbitol ou un saccharide, ou leurs dérivés alcoxylés, ou un ester de l'acide tartrique, citrique, ascorbique ou lactique.

5. Composition parfumante selon l'une des revendications 1 à 4, caractérisée en ce que l'émulsion aqueuse est constituée par

a. 2 à 20% d'agent filmogène,

b. 0,1 à 10% d'agent émulsifiant,

c. 0,1 à 5% de base parfumante,

d. 0,1 à 50% de base désodorisante ou antiperspirante active,

le restant étant représenté par de l'eau, des dissolvants inertes et/ou des excipients, le cas échéant, des agents germicides ou bactériostatiques.

6. Composition parfumante selon la revendication 1, caractérisée en ce que la base antiperspirante est constituée par un sel d'aluminium ou de zirconium.

7. Composition parfumante selon la revendication 6, caractérisée en ce que le sel d'aluminium ou zirconium est un hydroxychlorure d'aluminium ou de zirconium.

8. Dispositif ou article désodorisant ou antiperspirant destiné aux soins corporels caractérisé en ce qu'il contient une composition parfumante selon la revendication 1.

9. Dispositif ou article désodorisant ou antiperspirant selon la revendication 8 choisi parmi les crèmes, les sticks, les roll-on, les smooth-on, les aérosols ou les poudres.

Fig. 1

phase liquide     couche solide

Fig. 2

Fig. 3

phase liquide     couche solide